(19) Europäisches Patentamt
European Patent Office
Office européen des brevets

(11) Publication number : 0 379 070 B1

# (12) EUROPEAN PATENT SPECIFICATION

(45) Date of publication of patent specification : 23.06.93 Bulletin 93/25

(51) Int. Cl.$^5$ : **C07C 17/26,** C07C 41/06, C07C 19/08, C07C 43/12

(21) Application number : **90100541.3**

(22) Date of filing : **11.01.90**

## (54) Process for preparing chlorotrifluoroethylene telomers and telomers obtained thereby.

(30) Priority : **12.01.89 IT 1907889**

(43) Date of publication of application : **25.07.90 Bulletin 90/30**

(45) Publication of the grant of the patent : **23.06.93 Bulletin 93/25**

(84) Designated Contracting States : **BE DE FR GB NL**

(56) References cited :
**EP-A- 0 321 990**
**FR-A- 1 206 546**
**GB-A- 2 148 286**

(73) Proprietor : **AUSIMONT S.p.A.**
**Foro Buonaparte, 31**
**I-20121 Milano (IT)**

(72) Inventor : **Marraccini, Antonio, Dr.**
**3, Corso Riviera**
**I-28040 Dormellettto, Novara (IT)**
Inventor : **Pasquale, Antonio**
**8, Corso Milano**
**I-28100 Novara (IT)**
Inventor : **Vincenti, Marco, Dr.**
**185, Corso Francia**
**I-10139 Torino (IT)**

(74) Representative : **Barz, Peter, Dr. et al**
**Patentanwälte Dipl.-Ing. G. Dannenberg Dr. P. Weinhold, Dr. D. Gudel Dipl.-Ing. S. Schubert, Dr. P. Barz Siegfriedstrasse 8**
**W-8000 München 40 (DE)**

EP 0 379 070 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for preparing chlorotrifluoroethylene telomers. More particularly, the invention relates to a process for preparing telomers of chlorotrifluoroethylene with perhaloalkylfluoroxy compounds and the telomers obtained thereby.

US-A-4,577,044 describes a new type of chlorotrifluoroethylene telomers obtained by reacting chlorotrifluoroethylene with $CF_3OF$. The following telomeric species may be obtained: $F(CF_2\text{-}CFCl)_nF$, $CF_3O\text{-}(CF_2CFCl)_n\text{-}F$ and $CF_3O\text{-}(CF_2CFCl)_n\text{-}OCF_3$, wherein n usually ranges from 1 to 10 and the telomeric units ($CF_2$-CFCl) are distributed at random, i.e. they may be linked both in head-to-head and in head-to-tail fashion.

As regards the telomeric products which show a not too high degree of telomerization (as the products of the above US-patent) it is assumed that the nature of the end groups and the type of bond (oxygen-carbon or carbon-carbon) by which these groups are bound to the monomeric units significantly affect the physical and chemical properties of the telomers, in particular owing to different bond strengths, different sizes of the groups and differences in the steric hindrance thereof and in the flexibility of the bond type (oxygen-carbon or carbon-carbon).

Consequently, the telomers of chlorotrifluoroethylene with $CF_3OF$ cannot exhibit a wide range of properties as they can only possess $CF_3O$- and F- end groups. On the other hand, it is well known that an excellent property for a particular use of chlorotrifluoroethylene telomers may be of disadvantage for another application.

IT-A-23179 A/87 describes a process for preparing telomers of chlorotrifluoroethylene with perhaloalkylfluoroxy compounds of formula $R_y\text{-}CF_2OF$ in which $R_y$ is a perhaloalkyl radical having 1 to 10 carbon atoms and containing fluorine atoms or fluorine and chlorine atoms. With this process it is possible to obtain telomers having the following end groups:

- F;
- perhaloalkoxy groups, for example $R_y\text{-}CF_2\text{-}O\text{-}$ or $R_y$ -O-;
- perhaloalkyl groups,for example $R_y$- and $R_y\text{-}CF_2^-$.

Accordingly, telomer mixtures may be obtained which contain, inter alia, the following telomeric species (in the following the monomeric unit $CF_2$-CFCl is schematically represented by the letter M):

$$R_y\text{-}CF_2\text{-}O\text{-}(M)_n\text{-}F$$
$$R_y\text{-}CF_2\text{-}O\text{-}(M)_n\text{-}O\text{-}CF_2\text{-}R_y$$
$$R_y\text{-}CF_2\text{-}O\text{-}(M)_n\text{-}R_y$$
$$R_y\text{-}O\text{-}(M)_n\text{-}F$$
$$R_y\text{-}CF_2\text{-}(M)_n\text{-}F$$
$$F\text{-}(M)_n\text{-}F$$

By adjusting the operative conditions it is possible to obtain, in particular, mixtures of telomers having prevailingly F and perhaloalkoxy end groups or mixtures of telomers having, besides F and perhaloalkoxy end groups, a high proportion of perhaloalkyl end groups.

Thus it is possible to vary, within a wide range, the distribution and the nature of the end groups contained in the telomeric species, thereby obtaining a wide range of products having different physical and chemical properties and being suited for meeting various applicative requirements.

It has now been found that by adding elemental fluorine to the reactants (i.e. perhalofluoroxy compound and chlorotrifluoroethylene) it is possible, under certain conditions, to increase the proportion of telomers having one or two -F end groups.

This is surprising since fluorine does not exert any telogenic action on chlorotrifluoroethylene. In fact, it is known that the reaction of fluorine with chlorotrifluoroethylene yields an adduct which contains from one to two molecules of chlorotrifluoroethylene per molecule of $F_2$.

It has also surprisingly been found that again by adding elemental fluorine to the reactants (perhalofluoroxy compound and chlorotrifluoroethylene) it is possible, by following a different procedure, to obtain mixtures of telomers containing species with -Cl end groups (besides chlorine present in the telomeric end unit $-CF_2\text{-}CFCl\text{-}$).

Therefore, the range of obtainable products is considerably broadened by the present invention.

Mixtures of telomers having an increased content of -F end groups are of interest because a lower content of perhaloalkoxy (ether) end groups results in a lower compressibility of the fluid.

On the other hand, mixtures of telomers containing species with -Cl end groups exhibit a smaller variation of the viscosity as a function of the temperature.

Thus, it is one object of the present invention to provide a process for preparing telomers of chlorotrifluoroethylene with perhalofluoroxy compounds which allows to obtain, under certain conditions, an increased proportion of -F end groups.

Another object is to provide a process for preparing the above telomers which allows to obtain, by using a different procedure, mixtures of telomers containing species with -Cl end groups, which also means an increased Cl/F ratio.

Still another object is to provide new chlorotrifluoroethylene telomers having at one end a -Cl end group and at the other end a perhaloalkoxy or perhaloalkyl end group.

The first two objects are achieved by the process of the present invention for preparing chlorotrifluoroethylene telomers.

This process is characterized in that chlorotrifluoroethylene, elemental fluorine and a perhalofluoroxy compound of formula $CF_3OF$ or $R_x$-$CF_2OF$, in which $R_x$ is a straight or branched perhalogenated alkyl radical, a perhaloalkylmonoether radical or perhaloalkylpolyether radical having 1 to 10 carbon atoms and containing fluorine atoms or fluorine and chlorine atoms, are reacted at a temperature of from -100 to +40°C; the perhalofluoroxy compound and the elemental fluorine are fed to the reaction medium after dilution with a gas which is inert under the reaction conditions; and the ratio, in N liters/hour, of elemental fluorine to perhalofluoroxy compound ranges from 0.1:1 to 20:1, while the ratio

$$\frac{\text{perhalofluoroxy compound} + \text{elemental fluorine}}{\text{inert gas}}$$

in N liters/hour, ranges from 0.01:1 to 1:1.

The inert diluting gas may, for example, be nitrogen, argon, helium or gaseous chlorofluorocarbons selected from, e.g., 1,2-dichlorotetrafluoroethane and dichlorodifluoromethane. In order to carry out the reaction, preferably a stream of gaseous or liquid perhalofluoroxy compound and of gaseous fluorine, both diluted with the inert gas, is introduced into a reactor containing chlorotrifluoroethylene in the liquid state or dissolved in a solvent. More preferably, the perhalofluoroxy compound is fed in the gaseous state. If the reaction is carried out under superatmospheric pressure, it can be conducted at a higher temperature than the boiling point of chlorotrifluoroethylene (-27.9°C). If the reaction is carried out in solution, a chlorotrifluoroethylene solvent which is inert under the reaction conditions is used, preferably a chlorofluorocarbon such as, for example, 1,2-dichlorotetrafluoroethane, fluorotrichloromethane and dichlorodifluoromethane. Usually, the amount of chlorotrifluoroethylene in the solution ranges from 20 to 80% by weight.

When mixtures of telomers having an increased content of -F end groups are to be obtained, the following modalities are followed simultaneously:

- a not too high ratio of $F_2$ to perhalofluoroxy compound, in N liters/hours, is used;
- a rather low or very low ratio

  $$\frac{\text{perhalofluoroxy compound} + \text{elemental fluorine}}{\text{inert gas}}$$

  in N litres/hour, is used;
- conditions which reduce the amount of heat generated in the reaction medium and promote an effective dispersion thereof are utilized. Said conditions may be obtained by adopting one or both of the following measures:

  a) the reaction is carried out in a lower temperature range, provided said temperatures are still higher than the threshold temperature of the reaction between perhalofluoroxy compound and chlorotrifluoroethylene;

  b) telogen and fluorine are precooled before they are introduced; if necessary, the telogen is employed in the liquid state.

Another measure which helps to realize these conditions is effective stirring in the reactor.

To obtain telomer mixtures having an increased content of -F end groups, the following combination of modalities is preferably used:

a) the ratio $F_2$/perhalofluoroxy compound ranges from 0.1 to 10 and, particularly, from 0.1 to 3;

b) the ratio

$$\frac{\text{perhalofluoroxy compound} + \text{elemental fluorine}}{\text{inert gas}}$$

ranges from 0.01 to 0.50 and, particularly, from 0.03 to 0.35;

c) the reaction temperature ranges from -100 to -40°C and, particularly, from -80 to -60°C.

Within the range of said combination of modalities, the proportion of telomers having one or two -F end groups increases as the temperature decreases and the ratio

$$\frac{\text{perhalofluoroxy compound} + \text{elemental fluorine}}{\text{inert gas}}$$

decreases.

By following the procedure which promotes the formation of -F end groups, it is possible to obtain, for example, mixtures of telomers containing up to 45% by weight of F-$(M)_n$-F telomers having a F/Cl weight ratio equal to or higher than 0.6.

When mixtures of telomers containing species with -Cl end groups are to be obtained, the following measures are adopted simultaneously:

- a high ratio $F_2$/perhalofluoroxy compound is used;
- a low dilution of the perhalofluoroxy compound and of fluorine with the inert gas is used;
- conditions which increase the amount of heat generated in the reaction medium and do not promote the dispersion thereof are employed. For this purpose, a reaction temperature ranging from -75 to +40°C is used. Moderate stirring can be carried out, too.

To obtain mixtures of telomers containing species with -Cl end groups, the following combination of modalities is preferably employed:

a) a ratio $F_2$/fluoroxy compound ranging from 2 to 20 and, particularly, from 2 to 10, is used;

b) a ratio

$$\frac{\text{perhalofluoroxy compound} + \text{elemental fluorine}}{\text{inert gas}}$$

ranging from 0.3 to 1 is used;

c) a reaction temperature ranging from -75 to +40°C and, particularly, from -75 to -40°C, is used.

Within the range of the above combination of modalities the proportion of telomers having -Cl end groups increases with increasing temperature and with decreasing degree of dilution of perhalofluoroxy compound and fluorine with the inert gas.

By following the procedure which promotes the formation of -Cl groups it is possible to obtain, for example, more than 80% by weight of telomers having one or two Cl end groups and having a Cl/F weight ratio equal to or higher than 0.7.

According to another aspect of the process of the present invention, with the conditions under which it is possible to direct the telomerization reaction prevailingly towards the desired telomeric species, it furthermore is possible, by decreasing the amount of heat generated in the reaction medium and by promoting an effective dispersion thereof, to direct, within certain limits, the degree of telomerization towards low values. This may be achieved by using low flowrates of the telogen and of $F_2$ and a low temperature of the reaction medium and, optionally, by precooling of telogen and $F_2$.

Another measure which helps to obtain this result consists in carrying out intensive stirring in the reactor. Still another factor which favors this result is a very low ratio:

$$\frac{\text{perhalofluoroxy compound} + \text{elemental fluorine}}{\text{inert gas}}.$$

It is possible to obtain, for example, up to 80% and more of telomers having a value of n ranging from 1 to 6.

If the fluoroxy compound is introduced into the reactor in the liquid state, it is mixed with a liquid which is inert under the reaction conditions, in particular a chlorofluorocarbon, for example 1,2-dichlorotetrafluoroethane, fluorotrichloromethane and dichlorodifluoromethane, or it is conveyed, in the form of an aerosol, with the inert gas.

It is also possible to introduce into the reactor containing a solvent for chlorotrifluoroethylene a stream of elemental fluorine, inert gas and fluoroxy compound, either in the gaseous or in the liquid state, according to one of the feeding procedures described hereinbefore, and, separately, a stream of gaseous or liquid chlorotrifluoroethylene. In this case, chlorotrifluoroethylene preferably is fed in the liquid state.

It is assumed that the mechanism of the telogenic action of the fluoroxy compound partially passes through a homolytic breakage of the O-F bond according to the following scheme:

$$R_x\text{-}CF_2\text{-}OF \rightarrow R_x\text{-}CF_2\text{-}O^\circ + F^\circ$$

wherefore $R_x$-$CF_2$-O° and F° radicals can act as telomerization starters and terminators. It also is assumed that the above radicals can undergo further fragmentation and re-arrangement reactions with formation of other radicalic species, which in turn can act as telomerization starters and terminators. In the case of radicals $R_x$ containing Cl atoms it is assumed, moreover, that one or more chlorine atoms can be replaced by fluorine atoms.

Therefore, when starting from a perfluorofluoroxy compound, radicals $R^1$ derived from $R_x$ are formed wherein $R_x$ having at least two carbon atoms has lost one or more carbon atoms and/or $R_x$ having at least three carbon atoms has undergone a re-arrangement. More commonly, the radical $R^1$ is a radical $R^2$ which contains a smaller number of carbon atoms than $R_x$. For instance, when $R_x$ contains form 2 to 10 carbon atoms, $R^2$ contains from 1 to 9 carbon atoms.

When starting from a perhalofluoroxy compound containing chlorine, radicals $R^3$ derived from $R_x$ are formed, wherein $R_x$ having at least two carbon atoms has lost one or more carbon atoms and/or $R_x$ having at least three carbon atoms has undergone a re-arrangement and one or more chlorine atoms of $R_x$ have been substituted by fluorine atoms. More commonly, the radical $R^3$ is a radical $R^4$ which contains a smaller number of carbon atoms than $R_x$ (e.g., when $R_x$ contains from 2 to 10 carbon atoms, $R^4$ contains from 1 to 9 carbon

atoms) and in which one or more chlorine atoms have been substituted by fluorine atoms.

When starting from a perfluorofluoroxy compound, the obtainable telomers, depending on the operative conditions employed, have the following formulae (in each of the formulae the monomeric unit $CF_2$-CFCl is schematically represented by (M)):

$R_x$-$CF_2$-O-$(M)_n$-F (A)

$R_x$-$CF_2$-O-$(M)_n$-O-$CF_2$-$R_x$ (B)

$R_x$-$(M)_n$-F (C)

$R_x$-$(M)_n$-$R_x$ (D)

$R_x$-$CF_2$-O-$(M)_n$-$R_x$ (E)

$R_x$-O-$(M)_n$-F (F)

$R_x$-O-$(M)_n$-O-$CF_2$-$R_x$ (G)

$R_x$-O-$(M)_n$-$R_x$ (H)

$R_x$-$CF_2$-$(M)_n$-F (I)

$R_x$-$CF_2$-$(M)_n$-O-$CF_2$-$R_x$ (J)

$R_x$-$CF_2$-$(M)_n$-$R_x$ (K)

$R^1$-O-$(M)_n$-$OCF_2$-$R_x$ (L)

$R^1$-O-$(M)_n$-$R_x$ (N)

$R^1$-$(M)_n$-F (O)

$CF_3$-O-$(M)_n$-F (P)

F-$(M)_n$-F (Q)

$CF_3$-O-$(M)_n$-O-$CF_3$ (R)

$R_x$-$CF_2$-O-$(M)_n$-Cl (A')

$R_x$-$(M)_n$-Cl (C')

$R_x$-O-$(M)_n$-Cl (F')

$R_x$-$CF_2$-$(M)_n$-Cl (I')

$R^1$-$(M)_n$-Cl (O')

$CF_3$-O-$(M)_n$-Cl (P')

F-$(M)_n$-Cl (Q')

Cl-$(M)_n$-Cl (Q")

wherein $R_x$ is the same as defined above, n ranges from 1 to 20 and $R^1$ is a radical derived from $R_x$, in which $R_x$ having at least two carbon atoms has lost one or more carbon atoms and/or $R_x$ having at least 3 carbon atoms has undergone a re-arrangement.

Preferably, n ranges from 1 to 10.

The distribution of monomeric units -$CF_2$-CFCl- is statistical, i.e., said units can be linked both in head-to-head and in head-to-tail fashion.

The telomers (A'), (C'), (F'), (I'), (O') and (P') are new products.

In the obtained telomer mixtures small amounts of telomers having formulae different from the indicated ones may be present.

It is to be borne in mind that in the given telomer formulae, (M) may be either ($CF_2$-CFCl) or (CFCl -$CF_2$). Thus, two series of products correspond to telomers $R_x$-$CF_2$-O-$(M)_n$-F (A), which products are represented by:

1) $R_x$-$CF_2$-O-$(CF_2\text{-}CFCl)_n$-F ($A_1$)

in which the end group $R_x$-$CF_2$-O is bound to the $CF_2$ group while the end group F is bound to the group CFCl, independently of the linkage of the intermediate monomeric units when n is higher than 2.

2) $R_x$-$CF_2$-O-$(CFCl\text{-}CF_2)_n$-F ($A_2$)

in which the end group $R_x$-$CF_2$-O- is bound to the CFCl group while the end group F is bound to the group $CF_2$, independently of the linkage of the intermediate monomeric units when n is higher than 2.

When $R_x$ is a perhaloalkylpolyether radical, it preferably contains two oxygen atoms.

Preferably, $R_x$ contains from 1 to 4 carbon atoms.

The most preferred telogens are $CF_3OF$ and those of formula $R_x$-$CF_2OF$ wherein $R_x$ is a perfluoroalkyl radical containing from 1 to 4 carbon atoms (e.g. $CF_3$, $C_2F_5$ and $C_3F_7$).

When conditions are employed which increase the proportion of telomers having -F end groups, telomers of formulae (A), (B), (C), (D), (E), (F), (I), (J) and (Q) are obtained prevailingly.

When conditions are employed which favor mixtures of telomers containing species having -Cl end groups, prevailingly telomers (A), (B), (Q), (A'), (Q'), (Q") and, in smaller amounts, telomers (F), (P), (C), (E), (G), (F') and (C') are obtained.

The telomer mixtures obtained by means of the process of the present invention are useful in particular as hydraulic fluids and service fluids.

By fractionated distillation of the mixtures it is possible to obtain fractions prevailingly consisting of telomers

having a defined value of n.

Among the fluoroxy compounds utilizable in the process of the present invention, the following may be cited:

fluoroxy trifluoromethane
fluoroxy pentafluoroethane
1-fluoroxy heptafluoropropane
1-fluoroxy nonafluorobutane
1-fluoroxy 2-chlorotetrafluoroethane
1-fluoroxy 2,2 -dichlorotrifluoroethane
fluoroxy heptafluoroisopropane
fluoroxy nonafluoroisobutane
fluoroxy nonafluoro-tert-butane
1-fluoroxy-2-perfluoro-n-propoxy-hexafluoropropane
1-fluoroxy-2-perfluoromethoxy-hexafluoropropane
1-fluoroxy-2-perfluoroethoxy-hexafluoropropane and
1-fluoroxy-3-chlorohexafluoro-n-propane.

When $CF_3CF_2OF$ is used as telogen, the obtained telomer mixture may be composed, depending on the operative conditions, of the following species:

$F(M)_nF$ (Ia)
$F(M)_nOCF_2CF_3$ (IIa)
$CF_3CF_2O(M)_n\text{-}OCF_2CF_3$ (IIIa)
$CF_3(M)_n\text{-}F$ (IVa)
$CF_3(M)_n\text{-}OCF_2CF_3$ (Va)
$CF_3O(M)_n\text{-}OCF_2CF_3$ (VIa)
$CF_3CF_2\text{-}(M)_n\text{-}F$ (VIIa)
$CF_3CF_2(M)_n\text{-}OCF_2CF_3$ (VIIIa)
$CF_3O(M)_nF$ (IXa)
$CF_3\text{-}(M)_n\text{-}CF_3$ (Xa)
$F\text{-}(M)_n\text{-}Cl$ (I'a)
$Cl\text{-}(M)_nCl$ (I"a)
$CF_3CF_2O(M)_n\text{-}Cl$ (II'a)
$CF_3(M)_n\text{-}Cl$ (IV'a)
$CF_3O\text{-}(M)_n\text{-}Cl$ (IX'a)

The species (II'a), (IV'a) and (IX'a) are new telomers.

When starting from $CF_3\text{-}CF_2OF$ and directing the reaction towards the obtainment of telomers with an increased proportion of -F end groups, telomers (Ia), (IIa), (IIIa), (IVa), (Va) and (VIa) are obtained prevailingly.

When starting from $CF_3\text{-}CF_2OF$ and directing the reaction towards the obtainment of telomer mixtures containing species having -Cl end groups, telomers (Ia), (IIa), (IIIa), (IVa), (Va), (VIa), (IXa), (I'a), (I"a), (II'a), (IV'a) and (IX'a) are obtained prevailingly.

When $CF_3OF$ is used as telogen, the mixture of obtained telomers may be composed, depending on the operative conditions, of the following species:

$F(M)_nF$ (Ia)
$CF_3O(M)_nF$ (IXa)
$CF_3O(M)_nOCF_3$ (IIIb)
$F(M)_nCl$ (Ib)
$CF_3O(M)_nCl$ (IIb)
$Cl(M)_nCl$ (I"a)

Species (IIb) are new telomers.

When starting from $CF_3OF$ and directing the reaction towards the obtainment of telomers having an increased proportion of -F end groups, telomers (Ia) and (IXa) are prevailingly obtained.

When starting from $CF_3OF$ and directing the reaction towards the obtainment of telomer mixtures containing telomers having -Cl end groups, telomers (Ia), (Ib) and (IIb) are prevailingly obtained.

The main advantages of the present invention may be summarized as follows. It is possible to vary,over a wide range, the distribution and the nature of the end groups which are present in the obtained telomeric species and, within certain limits, the degree of telomerization, thereby being able to obtain a wide range of products having different physical and chemical properties and being capable of meeting various applicative requirements. In particular, it is possible to obtain mixtures of telomers having an increased content of -F end groups or telomer mixtures containing species having -Cl end groups.

The following examples are given for merely illustrative purposes.

Example 1

A flow of 0.32 N litres/hour of $CF_3$-$CF_2OF$, 0.88 N litres/hour of elemental fluorine and 30 N litres/hour of nitrogen was continuously bubbled into 204 g of liquid chlorotrifluoroethylene (CTFE), cooled to -72°C, in a glass reactor having a volume of 0.5 liter and being equipped with reflux cooler, thermometer and mechanical stirrer.

After 5 hours, the reaction was stopped and the unreacted CTFE was distilled off.
59 g of product, corresponding to a yield of about 36.9 g of telomers per N litre of gaseous $CF_3$-$CF_2OF$ were thus obtained.

By gas-chromatography, the composition was analyzed in terms of n. The product was further examined by gas-mass analysis by means of a capillary column SE-52. Furthermore, $^{19}F$ N.M.R. spectra (in $CDCl_3$) were recorded on a BRUKER instrument AM 300.

Compounds (Ia), (IIa), (IIIa), (IVa), (Va), (VIa), (VIIa), (VIIIa), (IXa) and (Xa) were determined to be present. Species (Ia), (IIa) and (IIIa) represented about 83% by weight of the mixture, with species (Ia) alone representing about 45%.

The proportion of species containing F end groups (Ia, IIa, IVa and IXa) was higher than 84% by weight of the total of species present in the aggregate.

In the product with n equal to 2, also the following compounds were present:

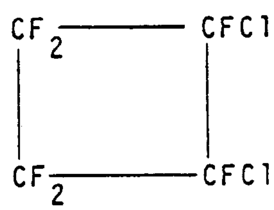

(I)

and

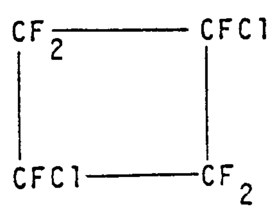

(II)

The distribution of the values of n as determined by gas-chromatography was as follows:

| value of n | area % |
|---|---|
| 1 | 14 |
| 2 | 57.4 |
| 3 | 6.5 |
| 4 | 9.1 |
| 5 | 6.7 |
| 6 | 3.7 |
| 7 | 1.7 |
| 8 | 0.7 |

Example 2

Following the procedure of example 1, 0.32 N litres/hour of $CF_3$-$CF_2$-OF, 0.88 N litres/hour of elemental fluorine and 3.0 N litres/hour of $N_2$ were bubbled into 187 g of CTFE, at -72°C, for 3 hours. After having distilled off the unreacted CTFE, 3.8 g of product were obtained.
Compounds Ia, IIa, IIIa, IVa, Va, VIa, IXa, I'a, I"a, II'a, IV'a and IX'a were present in the product. Species Ia, I'a, I"a and II'a represented about 90% of the mixtures. The telomers having a value of n ranging from 3 to 6 corresponded to 70% of the total. The elemental analysis of the product indicated that the Cl/F weight ratio was 0.7.

Example 3

Following the procedure of example 1, 0.85 N litres/hour of $CF_3$-$CF_2$OF. 0.35 N litres/hour of $F_2$ and 10 N litres/hour of $N_2$ were bubbled into 184 g of liquid CTFE, at -72°C, for 5 hours. After having distilled off the unreacted CTFE, 86.7 g of product were obtained. The molar yield with respect to $CF_3$-$CF_2$OF was about 99%, corresponding to about 20.4 g of telomers per liter of gaseous $CF_3$-$CF_2$OF.

The product was composed of the telomeric species Ia, IIa, IIIa, IVa, Va, VIa, VIIa, VIIIa, IXa and Xa. Species Ia, IIa and IIIa represented more than 80% of the mixture. The proportion of the species which contained F end groups (Ia, IIa, IVa and IXa) exceeded 79% by weight of the total of the species present in the aggregate.

The fraction with n equal to 2 also contained compounds (I) and (II), the formula of which is indicated in example 1.

Gas-chromatographic analyses revealed that the product consisted of telomers having values of n as indicated hereinbelow:

| value of n | area % |
|---|---|
| 1 | 19.1 |
| 2 | 22 |
| 3 | 15.4 |
| 4 | 25.8 |
| 5 | 12 |
| 6 | 4.4 |
| 7 | 1.4 |
| 8 | 0.3 |

Example 4

A comparative test (with respect to example 3) was carried out in the absence of elemental fluorine.

Following the procedure of example 1, 0.85 N litres/hour of $CF_3$-$CF_2$OF and 0 1 N litres/hour of nitrogen were bubbled into 184 g of CTFE at -72°C, for 5 hours. After removal of the unreacted CTFE, 75.2 g of product were obtained with a molar yield of about 80% with respect to $CF_3$-$CF_2$OF. Compounds Ia, IIa, IIIa, IVa, Va, VIa, VIIa, VIIIa, IXa, Xa were found to be present. Species Ia, IIa and IIIa represented about 80% of the mixture. The proportion of the species containing F end groups (Ia, IIa, IVa and IXa) was lower than 69 % by weight of the total of species present in the aggregate.

The telomers with values of n ranging from 2 to 6 corresponded to about 87% of the total.

Example 5

Following the procedure of example 1, 1.2 N litres/hour of $CF_3$OF, 0.6 N litres/hour of $F_2$ and 2.5 N litres/hour of $N_2$ were bubbled into 200 g of liquid CTFE,at -60°C, for 5 hours. After the unreacted CTFE was distilled off, 46 g of product were obtained, with a yield of about 7.7 g of telomers per liter of gaseous $CF_3$OF. Compounds Ia, Ib, IIb, I"a, IIIb and IXa were present.

## Claims

1. Process for preparing chlorotrifluoroethylene telomers, wherein chlorotrifluoroethylene, elemental fluorine and a perhalofluoroxy compound of formula $CF_3OF$ or $R_x$-$CF_2$-OF, in which $R_x$ represents a linear or branched perhaloalkyl, perhaloalkylmonoether or perhaloalkylpolyether radical having from 1 to 10 carbon atoms and containing fluorine atoms or fluorine and chlorine atoms, are reacted at a temperature of from -100 to +40°C and the perhalofluoroxy compound and the elemental fluorine are fed to the reaction medium after having been diluted with a gas which is inert under the reaction conditions, the ratio of fluorine to perhalofluoroxy compound, in N litres/hour, ranging from 0.1 to 20 and the ratio
$$\frac{\text{perhalofluoroxy compound} + \text{fluorine}}{\text{inert gas}}$$
in N litres/hour, ranging from 0.01 to 1.

2. Process according to claim 1, wherein a stream of liquid or, preferably, gaseous perhalofluoroxy compound and of gaseous fluorine, both diluted with an inert gas, is fed to a reactor containing chlorotrifluoroethylene in the liquid state or dissolved in a solvent, preferably a chlorofluorocarbon.

3. Process according to one or more of the preceding claims, wherein telomer mixtures having an increased content of -F end groups are obtained by employing a not too high ratio of fluorine to perhalofluoroxy compound, preferably from 0.1 to 10, a rather low or very low ratio
$$\frac{\text{perhalofluoroxy compound} + \text{fluorine}}{\text{inert gas}}$$
preferably from 0.01 to 0.50, and conditions which reduce the amount of heat generated in the reaction medium and promote a good dispersion thereof.

4. Process according to claim 3, wherein the reaction is carried out at a temperature ranging from -100 to -40°C.

5. Process according to any one of claims 1 and 2, wherein telomer mixtures containing species having -Cl end groups are obtained by employing a high ratio of fluorine to perhalofluoroxy compound, preferably from 2 to 20, a low dilution of fluorine and perhalofluoroxy compound with the inert gas, the ratio
$$\frac{\text{perhalofluoroxy compound} + \text{fluorine}}{\text{inert gas}}$$
preferably being from 0.3 to 1, and conditions which increase the amount of heat generated in the reaction medium and do not promote the dispersion thereof.

6. Process according to claim 5, wherein the reaction is carried out at a temperature ranging from -75° to +40°C.

7. Process according to one or more of the preceding claims, wherein $R_x$ contains two oxygen atoms when it is a perhaloalkylpolyether radical.

8. Process according to one or more of the preceding claims, wherein $R_x$ contains from 1 to 4 carbon atoms.

9. Process according to one or more of the preceding claims, wherein the perhalofluoroxy compound is $CF_3OF$ or a compound of formula $R_x$-$CF_2OF$ wherein $R_x$ is a perfluoroalkyl radical containing from 1 to 4 carbon atoms.

10. Chlorotrifluoroethylene telomers having one of the following formulae:

$R_x$-$CF_2$-O-$(M)_n$-Cl (A')

$R_x$-$(M)_n$-Cl (C')

$R_x$-O-$(M)_n$-Cl (F')

$R_x$-$CF_2$-$(M)_n$-Cl (I')

$R^1$-$(M)_n$-Cl (O')

wherein $R_x$ is a linear or branched perhaloalkyl, perhaloalkylmonoether or perhaloalkylpolyether radical having from 1 to 10 carbon atoms and containing fluorine atoms or fluorine and chlorine atoms; M represents $CF_2$-CFCl; n ranges from 1 to 20; and $R^1$ represents a radical derived from $R_x$ in which $R_x$ having at least two carbon atoms has lost one or more carbon atoms and/or $R_x$ having at least three carbon atoms has undergone a re-arrangement.

11. Chlorotrifluoroethylene telomers having one of the following formulae:

$CF_3CF_2\text{-}O\text{-}(M)_n\text{-}Cl$ (II'a)

$CF_3\text{-}(M)_n\text{-}Cl$ (IV'a)

$CF_3O\text{-}(M)_n\text{-}Cl$ (IX'a)

wherein M and n are defined as in claim 10.

12. Mixtures of chlorotrifluoroethylene telomers containing telomers of the following formulae:

$R_x\text{-}CF_2\text{-}O\text{-}(M)_n\text{-}F$ (A)

$R_x\text{-}CF_2\text{-}O\text{-}(M)_n\text{-}O\text{-}CF_2\text{-}R_x$ (B)

$F\text{-}(M)_n\text{-}F$ (Q)

$R_x\text{-}CF_2\text{-}O\text{-}(M)_n\text{-}Cl$ (A')

$CF_3\text{-}O\text{-}(M)_n\text{-}Cl$ (P')

$F\text{-}(M)_n\text{-}Cl$ (Q')

$Cl\text{-}(M)_n\text{-}Cl$ (Q")

$R_x\text{-}(M)_n\text{-}Cl$ (C')

$R_xO(M)_nCl$ (F')

wherein $R_x$, M and n are defined as in claim 10.

13. Telomers or telomer mixtures according to any one of claims 10 to 12, wherein $R_x$ contains two oxygen atoms when it is a perhaloalkylpolyether radical.

14. Telomers or telomer mixtures according to any one of claims 10 to 13, wherein $R_x$ contains from 1 to 4 carbon atoms.

15. Telomers or telomer mixtures according to any one of claims 10 to 12 and 14, wherein $R_x$ is a perfluoroalkyl radical.

16. Mixtures of chlorotrifluoroethylene telomers containing telomers of the following formulae:

$F(M)_nF$ (Ia)

$F(M)_nOCF_2CF_3$ (IIa)

$CF_3CF_2O(M)_n\text{-}OCF_2CF_3$ (IIIa)

$CF_3(M)_n\text{-}F$ (IVa)

$CF_3(M)_n\text{-}OCF_2CF_3$ (Va)

$CF_3O(M)_n\text{-}OCF_2CF_3$ (VIa)

$CF_3CF_2\text{-}(M)_n\text{-}F$ (VIIa)

$CF_3CF_2(M)_n\text{-}OCF_2CF_3$ (VIIIa)

$CF_3O(M)_nF$ (IXa)

$CF_3\text{-}(M)_n\text{-}CF_3$ (Xa)

$F\text{-}(M)_n\text{-}Cl$ (I'a)

$Cl\text{-}(M)_n\text{-}Cl$ (I"a)

$CF_3CF_2O(M)_n\text{-}Cl$ (II'a)

$CF_3\text{-}(M)_n\text{-}Cl$ (IV'a)

$CF_3O\text{-}(M)_n\text{-}Cl$ (IX'a)

or telomers of the following formulae:

$F(M)_nF$ (Ia)

$CF_3O(M)_nF$ (IXa)

$CF_3O(M)_nOCF_3$ (IIIb)

$F(M)_nCl$ (Ib)

$CF_3O(M)_nCl$ (IIb)

$Cl(M)_nCl$ (I"a)

wherein M and n are defined as in claim 10.

**Patentansprüche**

1. Verfahren zur Herstellung von Chlortrifluorethylen-Telomeren, bei dem Chlortrifluorethylen, elementares Fluor und eine Perhaloenfluoroxyverbindung der Formel $CF_3OF$ oder $R_x\text{-}CF_2\text{-}OF$, in welcher $R_x$ einen linearen oder verzweigten Perhalogenalkyl-, Perhalogenalkylmonoether- oder Perhalogenalkylpolyether-

Rest mit 1 bis 10 Kohlenstoffatomen, der Fluoratome oder Fluor- und Chloratome enthält, darstellt, bei einer Temperatur von -100 bis +40°C umgesetzt werden und die Perhalogenfluoroxyverbindung und das elementare Fluor dem Reaktionsmedium nach Verdünnung mit einem Gas, das unter den Reaktionsbedingungen inert ist, zugeführt werden, wobei das Verhältnis von Fluor zu Perhalogenfluoroxyverbindung, in Normal-Litern/Stunde, im Bereich von 0,1 bis 20 liegt und das Verhältnis

$$\frac{\text{Perhalogenfluoroxyverbindung} + \text{Fluor}}{\text{Inertgas}}$$

in Normal-Litern/Stunde, im Bereich von 0,01 bis 1 liegt.

2. Verfahren nach Anspruch 1, bei welchem ein Strom von flüssiger oder, vorzugsweise, gasförmiger Perhalogenfluoroxyverbindung und gasförmigem Fluor, beide mit einem Inertgas verdünnt, einem Reaktor, der Chlortrifluorethylen im flüssigen Zustand oder in einem Lösungsmittel, vorzugsweise einem Chlorfluorkohlenstoff, gelöst enthält, zugeführt wird.

3. Verfahren nach einem oder mehreren der vorangehenden Ansprüche, bei welchem Telomer-Mischungen mit einem erhöhten Gehalt an F-Endgruppen durch Verwendung eines nicht zu hohen Verhältnisses von Fluor zu Perhalogenfluoroxyverbindung, vorzugsweise von 0,1 bis 10, eines relativ niedrigen oder sehr niedrigen Verhältnisses

$$\frac{\text{Perhalogenfluoroxyverbindung} + \text{Fluor}}{\text{Inertgas}}$$

vorzugsweise von 0,01 bis 0,50, und von Bedingungen, die die im Reaktionsmedium erzeugte Wärmemenge vermindern und eine gute Dispergierung derselben fördern, erhalten werden.

4. Verfahren nach Anspruch 3, bei welchem die Reaktion bei einer Temperatur im Bereich von -100 bis -40°C durchgeführt wird.

5. Verfahren nach irgendeinem der Ansprüche 1 und 2, bei welchem Telomer-Mischungen, die Spezies mit Cl-Endgruppen enthalten, durch Verwendung eines hohen Verhältnisses von Fluor zu Perhalogenfluoroxyverbindung, vorzugsweise von 2 bis 20, einer niedrigen Verdünnung von Fluor und Perhalogenfluoroxyverbindung mit dem Inertgas, wobei das Verhältnis

$$\frac{\text{Perhalogenfluoroxyverbindung} + \text{Fluor}}{\text{Inertgas}}$$

vorzugsweise 0,3 bis 1 beträgt, und von Bedingungen, die die im Reaktionsmedium erzeugte Wärmemenge erhöhen und die Dispergierung derselben nicht fördern, erhalten werden.

6. Verfahren nach Anspruch 5, bei welchem die Reaktion bei einer Temperatur im Bereich von -75 bis +40°C durchgeführt wird.

7. Verfahren nach irgendeinem der vorangehenden Ansprüche, bei welchem $R_x$ zwei Sauerstoffatome enthält, wenn es für einen Perhalogenalkylpolyetherrest steht.

8. Verfahren nach einem oder mehreren der vorangehenden Ansprüche, bei welchem $R_x$ 1 bis 4 Kohlenstoffatome enthält.

9. Verfahren nach einem oder mehreren der vorangehenden Ansprüche, bei welchem die Perhalogenfluoroxyverbindung $CF_3OF$ oder eine Verbindung der Formel $R_x$-$CF_2OF$, in welcher $R_x$ für einen Perfluoralkylrest, der 1 bis 4 Kohlenstoffatome enthält, steht, ist.

10. Chlortrifluorethylen-Telomere mit einer der folgenden Formeln:

$$R_x\text{-}CF_2\text{-}O\text{-}(M)_n\text{-}Cl \quad (A')$$

$$R_x\text{-}(M)_n\text{-}Cl \quad (C')$$

$$R_x\text{-}O\text{-}(M)_n\text{-}Cl \quad (F')$$

$$R_x\text{-}CF_2\text{-}(M)_n\text{-}Cl \quad (I')$$

$$R^1\text{-}(M)_n\text{-}Cl \quad (O')$$

worin

$R_x$ ein linearer oder verzweigter Perhalogenalkyl-, Perhalogenalkylmonoether-oder Perhalogenalkylpolyether -Rest mit 1 bis 10 Kohlenstoffatomen, der Fluoratome oder Fluor- und Chlor-Atome enthält, ist;

M für $CF_2$-CFCl steht;

n im Bereich von 1 bis 20 liegt; und

$R^1$ einen Rest darstellt, der von $R_x$ abgeleitet ist, wobei $R_x$ mit wenigstens zwei Kohlenstoffatomen ein oder mehr Kohlenstoffatome verloren hat und/oder $R_x$ mit wenigstens drei Kohlenstoffatomen sich umgelagert hat.

**11.** Chlortrifluorethylen-Telomere mit einer der folgenden Formeln:

$CF_3CF_2$-O-$(M)_n$-Cl (II'a)

$CF_3$-$(M)_n$-Cl (IV'a)

$CF_3O$-$(M)_n$-Cl (IX'a)

worin M und n wie in Anspruch 10 definiert sind.

**12.** Mischungen von Chlortrifluorethylen-Telomeren, die Telomere der folgenden Formeln enthalten:

$R_x$-$CF_2$-O-$(M)_n$-F (A)

$R_x$-$CF_2$-O-$(M)_n$-O-$CF_2$-$R_x$ (B)

F-$(M)_n$-F (Q)

$R_x$-$CF_2$-O-$(M)_n$-Cl (A')

$CF_3$-O-$(M)_n$-Cl (P')

F-$(M)_n$-Cl (Q')

Cl-$(M)_n$-Cl (Q")

$R_x$-$(M)_n$-Cl (C')

$R_xO(M)_n$-Cl (F')

worin $R_x$, M und n wie in Anspruch 10 definiert sind.

**13.** Telomere oder Telomermischungen nach irgendeinem der Ansprüche 10 bis 12, worin $R_x$ zwei Sauerstoffatome enthält, wenn es für einen Perhalogenalkylpolyetherrest steht.

**14.** Telomere oder Telomermischungen nach irgendeinem der Ansprüche 10 bis 13, worin $R_x$ 1 bis 4 Kohlenstoffatome enthält.

**15.** Telomere oder Telomermischungen nach irgendeinem der Ansprüche 10 bis 12 und 14, worin $R_x$ ein Perfluoralkylrest ist.

**16.** Mischungen von Chlortrifluorethylen-Telomeren, die Telomere mit den folgenden Formeln:

F $(M)_n$F (Ia)

$F(M)_nOCF_2CF_3$ (IIa)

$CF_3CF_2O(M)_n$-$OCF_2CF_3$ (IIIa)

$CF_3(M)_n$-F (IVa)

$CF_3(M)_n$-$OCF_2CF_3$ (Va)

$CF_3O(M)_n$-$OCF_2CF_3$ (VIa)

$CF_3CF_2(M)_n$-F (VIIa)

$CF_3CF_2(M)_n$-$OCF_2CF_3$ (VIIIa)

$CF_3O(M)_nF$ (IXa)

$CF_3$-$(M)_n$-$CF_3$ (Xa)

F-$(M)_n$-Cl (I'a)

Cl-$(M)_n$-Cl (I"a)

$CF_3CF_2O(M)_n$-Cl (II'a)

$CF_3$-$(M)_n$-Cl (IV'a)

$CF_3O$-$(M)_n$-Cl (IX'a)

oder Telomere mit den folgenden Formeln:

$F(M)_nF$ (Ia)

$CF_3O(M)_nF$ (IXa)

$CF_3O(M)_nOCF_3$ (IIIb)

$F(M)_nCl$ (Ib)

$CF_3O(M)_nCl$ (IIb)

$Cl(M)_nCl$ (I"a)

enthalten, worin M und n wie in Anspruch 10 definiert sind.

## Revendications

1. Procédé de préparation de télomères de chlorotrifluoroéthylène, caractérisé en ce que du chlorotrifluoroéthylène, du fluor élémentaire et un composé perhalofluoroxy de formule $CF_3OF$ ou $R_x$-$CF_2OF$, dans lequel Rx est un radical alkyle perhalogéné, linéaire ou ramifié, un radical perhaloalkylmonoéther ou perhaloalkylpolyéther comportant 1 à 10 atomes de carbone et contenant des atomes de fluor ou des atomes de fluor et de chlore sont amenés à réagir à une température comprise entre -100 et +40°C, le composé perhalofluoroxy et le fluor élémentaire étant alimentés dans le milieu réactionnel après dilution avec un gaz qui est inerte dans les conditions réactionnelles, et le rapport exprimé en N litres/heure de fluor élémentaire-composé perhalofluoroxy est compris entre 0,1/1 et 20/1, alors que le rapport:
$$\frac{\text{composé perhalofluoroxy} + \text{fluor}}{\text{gaz inerte}}$$
exprimé en N litres/heure est compris entre 0,01/1 et 1/1.

2. Procédé selon la revendication 1, caractérisé en ce qu'un courant de composé perhalofluoroxy liquide ou, de préférence, gazeux et de fluor gazeux, tous les deux dilués dans un gaz inerte, est introduit dans le réacteur contenant un chlorotrifluoroéthylène à l'état liquide ou dissous dans un solvant, de préférence un dérivé chlorofluorocarboné.

3. Procédé selon une ou plusieurs des revendications précédentes, dans lequel les mélanges de télomères présentant une teneur accrue en groupes terminaux -F sont obtenus par emploi d'un rapport pas trop élevé fluor/composé perhalofluoroxy, de préférence un rapport compris entre 0,1 et 10, un rapport plutôt faible ou très faible:
$$\frac{\text{composé perhalofluoroxy} + \text{fluor}}{\text{gaz inerte}}$$
de préférence compris entre 0,1 et 0,5, et des conditions qui réduisent la quantité de chaleur générée dans le milieu réactionnel et promeuvent une bonne dispersion de ce milieu.

4. Procédé selon la revendication 3, caractérisé en ce que la réaction est mise en oeuvre à une température comprise entre -100 et -40°C.

5. Procédé selon l'une quelconque des revendications 1 et 2, caractérisé en ce que les mélanges de télomères contenant des types de télomères présentant des groupes terminaux -Cl sont obtenus par emploi d'un rapport élevé fluor/composé perhalofluoroxy, de préférence compris entre 2 et 20, une faible dilution de fluor et du composé perhalofluoroxy dans le gaz inerte, le rapport:
$$\frac{\text{composé perhalofluoroxy} + \text{fluor}}{\text{gaz inerte}}$$
étant de préférence compris entre 0,3 et 1, et les conditions qui augmentent la quantité de chaleur produite dans le milieu réactionnel et qui ne promeuvent pas la dispersion de ce milieu.

6. Procédé selon la revendication 5, caractérisé en ce que la réaction est mise en oeuvre à une température comprise entre -75 et +40°C.

7. Procédé selon une ou plusieurs des revendications précédentes, caractérisé en ce que $R_x$ contient deux atomes d'oxygène lorsqu'il consiste en un radical perhaloalkylpolyéther.

8. Procédé selon une ou plusieurs des revendications précédentes, caractérisé en ce que $R_x$ contient de 1 à 4 atomes de carbone.

9. Procédé selon une ou plusieurs des revendications précédentes, caractérisé en ce que le composé perhalofluoroxy est $CF_3OF$ ou un composé de formule $R_x$-$CF_2OF$ dans lequel $R_x$ est un radical perfluoroalkyle contenant de 1 à 4 atomes de carbone.

10. Télomères de chlorotrifluoroéthylène présentant une des formules suivantes:

$R_x$-$CF_2$-O-$(M)_n$-Cl (A')

$R_x$-$(M)_n$-Cl (C')

$R_x$-O-$(M)_n$-Cl (F')

$R_x$-$CF_2$-$(M)_n$-Cl (I')

$R^1$-$(M)_n$-Cl (O')

dans lesquelles:

- $R_x$ est un radical alkyle perhalogéné, linéaire ou ramifié, un radical perhaloalkylmonoéther ou perhaloalkylpolyéther comportant 1 à 10 atomes de carbone et contenant des atomes de fluor ou des atomes de fluor et de chlore;
- M représente $CF_2$-CFCl;
- n est compris entre 1 et 20; et
- $R^1$ représente un radical dérivant de $R_x$ dans lequel $R_x$, présentant au moins deux atomes de carbone, a perdu un ou plusieurs atomes de carbone et/ou $R_x$, présentant au moins trois atomes de carbone, a subi un ré-arrangement.

**11.** Télomères de chlorotrifluoroéthylène présentant l'une des formules suivantes:

$CF_3CF_2$-O-$(M)_n$-Cl (II'a)

$CF_3$-$(M)_n$-Cl (IV'a)

$CF_3O$-$(M)_n$-Cl (IX'a)

dans lesquelles M et n sont tels que définis dans la revendication 10.

**12.** Mélanges de télomères de chlorotrifluoroéthylène contenant des télomères de formules suivantes:

$R_x$-$CF_2$-O-$(M)_n$-F (A)

$R_x$-$CF_2$-O-$(M)_n$-O-$CF_2$-$R_x$ (B)

F-$(M)_n$-F (Q)

$R_x$-$CF_2$-O-$(M)_n$-Cl (A')

$CF_3$-O-$(M)_n$-Cl (P')

F-$(M)_n$-Cl (Q')

Cl-$(M)_n$-Cl (Q")

$R_x$-$(M)_n$-Cl (C')

$R_xO(M)_n$-Cl (F')

dans lesquelles $R_x$, M et n sont tels que définis dans la revendication 10.

**13.** Télomères ou mélanges de télomères selon l'une quelconque des revendications 10 à 12, dans lesquels $R_x$ contient deux atomes d'oxygène lorsqu'il consiste en un radical perhaloalkylpolyéther.

**14.** Télomères ou mélanges de télomères selon l'une quelconque des revendications 10 à 13, caractérisé en ce que $R_x$ contient de 1 à 4 atomes de carbone.

**15.** Télomères ou mélanges de télomères selon l'une quelconque des revendications 10 à 12 et 14, dans lesquels $R_x$ est un radical perfluoroalkyle.

**16.** Mélanges de télomères de chlorotrifluoroéthylène contenant des télomères de formules suivantes:

$F(M)_nF$ (Ia)

$F(M)_nOCF_2CF_3$ (IIa)

$CF_3CF_2(M)_n$-$OCF_2CF_3$ (IIIa)

$CF_3(M)_n$-F (IVa)

$CF_3(M)_n$-$OCF_2CF_3$ (Va)

$CF_3O(M)_n$-$OCF_2CF_3$ (VIa)

$CF_3CF_2$-$(M)_n$-F (VIIa)

$CF_3CF_2(M)_n$-$OCF_2CF_3$ (VIIIa)

$CF_3O(M)_nF$ (IXa)

$CF_3$-$(M)_n$-$CF_3$ (Xa)

F-$(M)_n$-Cl (I'a)

Cl-$(M)_n$-Cl (I"a)

$CF_3CF_2O(M)_n$-Cl (II'a)

$CF_3(M)_n$-Cl (IV'a)

$CF_3O$-$(M)_n$-Cl (IX'a)

ou des télomères de formules suivantes:

$F(M)_nF$ (Ia)

$CF_3O(M)_nF$ (IXa)

$CF_3O(M)_nOCF_3$ (IIIb)

$F(M)_nCl$ (Ib)

$CF_3O(M)_nCl$ (IIb)

$$Cl(M)_nCl \qquad (I''a)$$

dans lesquelles M et n sont tels que définis dans la revendication 10.